(19) Europäisches Patentamt
European Patent Office
Office européen des brevets

(11) Veröffentlichungsnummer: **0 120 238**
**B1**

(12) **EUROPÄISCHE PATENTSCHRIFT**

(45) Veröffentlichungstag der Patentschrift: **15.10.86**

(51) Int. Cl.⁴: **A 01 N 31/04**

(21) Anmeldenummer: **84101203.2**

(22) Anmeldetag: **07.02.84**

# (54) Mikrobizides Mittel zum Schutz technischer Materialien.

(30) Priorität: **19.02.83 DE 3305834**

(43) Veröffentlichungstag der Anmeldung: **03.10.84 Patentblatt 84/40**

(45) Bekanntmachung des Hinweises auf die Patenterteilung: **15.10.86 Patentblatt 86/42**

(84) Benannte Vertragsstaaten: **BE CH DE FR GB IT LI NL SE**

(56) Entgegenhaltungen:
**DE-A-2 942 618**
**US-A-3 636 161**

(73) Patentinhaber: **BAYER AG, Konzernverwaltung RP Patentabteilung, D-5090 Leverkusen 1 Bayerwerk (DE)**

(72) Erfinder: **Krüger, Bernd- Wieland, Dr., Sillerstrasse 49, D-5600 Wuppertal 11 (DE)**
Erfinder: **Priesnitz, Uwe, Dr., Severinstrasse 58, D-5650 Solingen 1 (DE)**
Erfinder: **Jäger, Gerhard, Dr., Gellertstrasse 18, D-5090 Leverkusen 1 (DE)**
Erfinder: **Paulus, Wilfried, Dr., deswatinesstrasse 90, D-4150 Krefeld 1 (DE)**
Erfinder: **Genth, Hermann, Dr., Am Heckerhof 60, D-4150 Krefeld 1 (DE)**

EP 0 120 238 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft mikrobizide Mittel zum Schutz technischer Materialien vor Schädigung oder Zerstörung durch Mikroorganismen.

Aus der DE—A 29 42 618 sind spezielle Cyclopropanderivate bekannt, die sich zur Bekämpfung von Schädlingen in der Landwirtschaft eignen. Aus der US—A 36 36 161 ist ein Verfahren zur Herstellung von 2,2-Dihalogencyclopropylcarbinolen bekannt, die sich zur Herstellung von beispielsweise Fungiziden eignen.

Es wurde ein mikrobizides Mittel zum Schutz technischer Materialien gefunden, das Cyclopropylmethyl(en)ether der Formel

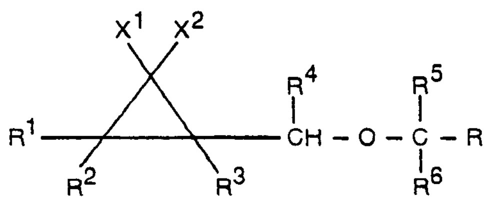

(I),

in der

$X^1$ und $X^2$ gleich oder verschieden sind und für Halogen stehen,

$R^1$ für Wasserstoff oder Alkyl steht,

$R^2$ und $R^3$ gleich oder verschieden sind und für Wasserstoff, Alkyl, gegebenenfalls substituiertes Aryl, Halogenalkyl, Hetaryl-alkyl, Alkoxyalkyl, Alkylthioalkyl, gegebenenfalls substituiertes Alkenoxyalkyl, gegebenenfalls substituiertes Alkinoxyalkyl oder für (Di)Alkylaminoalkyl stehen,

$R^4$ für Wasserstoff oder Alkyl steht,

$R^5$ und $R^6$ gleich oder verschieden sind und für Wasserstoff oder gegebenenfalls substituierte Reste aus der Reihe Alkyl, Aryl und Aralkyl stehen und

R für einen Trihalogenalkenyl-, Alkinyl-, oder Jodalkinyl-Rest steht,

enthält.

Die erfindungsgemäßen mikrobiziden Mittel haben eine hervorragende Wirkung zum Schutz technischer Materialien vor Schädigung oder Zerstörung durch Mikroorganismen.

Als Alkyl $R^1$, $R^2$, $R^3$ und $R^4$ sowie als gegebenenfalls substituiertes Alkyl $R^5$ und $R^6$ kann geradkettiges oder verzweigtes Alkyl mit 1 bis 20, vorzugsweise 1 bis 10, insbesondere bevorzugt 1 bis 6 und besonders bevorzugt 1 bis 4 Kohlenstoffatomen stehen. Beispielsweise seien gegebenenfalls substituiertes Methyl, Ethyl, n- und Isopropyl, n-, iso-, sec- und tert.-Butyl genannt.

Jodalkinyl R und Alkinyl R enthalten im Alkinylteil vorzugsweise 2 bis 5, insbesondere 2 bis 4 Kohlenstoffatome. Beispielsweise seien Ethinyl, Propinyl-(1), Propinyl-(2) und Butinyl-(3) und die entsprechenden Jod-Derivate genannt.

Als gegebenenfalls substituiertes Aryl $R^2$, $R^3$, $R^5$ und $R^6$ steht Aryl mit vorzugsweise 6 oder 10 Kohlenstoffatomen im Arylteil. Beispielhaft seien gegebenenfalls substituiertes Phenyl oder Naphthyl, insbesondere Phenyl, genannt.

Als gegebenenfalls substituiertes Aralkyl $R^5$ und $R^6$ steht gegebenenfalls im Arylteil und/oder Alkylteil substituiertes Aralkyl mit vorzugsweise 6 oder 10, insbesondere 6, Kohlenstoffatomen im Arylteil und vorzugsweise 1 bis 4, insbesondere 1 oder 2, Kohlenstoffatome im Alkylteil, wobei der Alkylteil geradkettig oder verzweigt sein kann. Beispielhaft seien gegebenfalls substituiertes Benzyl und Phenyl-ethyl genannt.

Halogenalkyl $R^2$ und $R^3$ enthalten im Alkylteil vorzugsweise 1 bis 4 Kohlenstoffatomen, insbesondere 1 oder 2 Kohlenstoffatome, und vorzugsweise 1 bis 5, insbesondere 1 bis 3, Halogenatome, wobei die Halogenatome gleich oder verschieden sind und als Halogenatome, vorzugsweise Chlor oder Brom stehen. Beispielhaft seien Chlormethyl oder Brommethyl genannt.

Alkoxyalkyl und Alkylthioalkyl $R^2$ und $R^3$ enthalten je Alkylteil vorzugsweise 1 bis 4 Kohlenstoffatome, insbesondere 1 oder 2 Kohlenstoffatome, wobei der Alkylteil geradkettig oder verzweigt sein kann. Beispielhaft seien Methoxymethyl, Methoxyethyl, Ethoxymethyl, Ethoxyethyl, Methylthiomethyl, Methylthioethyl, Ethylthiomethyl und Ethylthioethyl genannt.

Als gegebenenfalls substituiertes Alkenoxyalkyl $R^2$ oder $R^3$ steht gegebenenfalls im Alkenoxyteil und/oder Alkylteil substituiertes Alkenoxyalkyl mit vorzugsweise 2 bis 5, insbesondere 2 bis 4 Kohlenstoffatomen im Alkenoxyteil und vorzugsweise 1 bis 4, insbesondere 1 oder 2 Kohlenstoffatome, im Alkylteil, wobei der Alkylteil geradkettig oder verzweigt sein kann. Beispielhaft seien gegebenenfalls substituiertes Propenyloxymethyl, Butenyloxymethyl und Methyl-propenyloxymethyl genannt.

Als gegebenenfalls substituiertes Alkinoxyalkyl $R^2$ und $R^3$ steht gegebenenfalls im Alkinoxyteil und/oder Alkylteil substituiertes Alkinoxyalkyl mit vorzugsweise 2 bis 5, insbesondere 2 bis 4 Kohlenstoffatomen im Alkinoxyteil und vorzugsweise 1 bis 4, insbesondere 1 oder 2 Kohlenstoffatome im Alkylteil, wobei der Alkylteil geradkettig oder verzweigt sein kann. Beispielhaft seien gegebenenfalls substituiertes Propinyloxymethyl, Butinyloxymethyl und Methylpropinyloxy genannt.

(Di)Alkylamino-alkyl $R^2$ und $R^3$ enthalten im Alkylteil vorzugsweise 1 bis 4 Kohlenstoffatome,

insbesondere 1 oder 2 Kohlenstoffatome. Beispielhaft seien (Di)Methylamino-methyl, (Di)Methylaminoethyl, (Di)Ethylamino-methyl und (Di)Ethylamino-ethyl genannt. (Di)Alkylamino-alkyl bedeutet Mono- oder Dialkylamino-alkyl.

Hetaryl-alkyl $R^2$ und $R^3$ enthalten im Hetarylteil 5 bis 7 gliedrige vorzugsweise 5 oder 6 gliedrige Ringe mit vorzugsweise 1 bis 3, insbesondere 1 oder 2 gleichen oder verschiedenen Heteroatomen, wie Sauerstoff, Schwefel und/oder Stickstoff, vorzugsweise Stickstoff und im Alkylteil 1 bis 4 Kohlenstoffatome, insbesondere 1 oder 2 Kohlenstoffatome. Beispielhaft seien 1-Pyrazolylmethyl, 1-Imidazolylmethyl und 1-(1,2,4-Triazolyl)methyl genannt.

Halogen bedeutet Fluor, Chlor, Brom oder Jod, vorzugsweise Fluor, Chlor oder Brom, besonders bevorzugt Chlor oder Brom.

Trihalogenalkenyl R enthält im Alkenylteil geradkettiges oder verzweigtes Alkenyl mit vorzugsweise 2 bis 5, insbesondere 2 bis 4, Kohlenstoffatomen. Die Halogenatome können gleich oder verschieden sein und stehen vorzugsweise für Fluor, Chlor, Brom und/oder Jod, insbesondere für Chlor, Brom und/oder Jod. Beispielhaft seien Trijodethenyl, Dibrom-jodethenyl und Dichlor-jodethenyl genannt.

Die in den Definitionen von $R^2$, $R^3$, $R^5$ und $R^6$ genannten substituierten Reste können einen oder mehrere, vorzugsweise 1 bis 3, insbesondere 1 oder 2 gleiche oder verschiedene Substituenten tragen. Als Substituenten seien beispielhaft aufgeführt: Alkyl mit vorzugsweise 1 bis 4, insbesondere 1 oder 2 Kohlenstoffatome, wie Methyl, Ethyl, n- und iso-Propyl; Alkoxy mit vorzugsweise 1 bis 4, insbesondere 1 oder 2 Kohlenstoffatomen, wie Methoxy, Ethoxy, n-und iso-Propoxy; Alkylthio mit vorzugsweise 1 bis 4, insbesondere 1 oder 2 Kohlenstoffatomen, wie Methylthio, Ethylthio, n- und iso-Propylthio; Halogen vorzugsweise Fluor, Chlor, Brom und Jod, insbesondere Chlor, Brom und Jod; Cyano und Nitro; Halogenalkyl, Halogenalkoxy und Halogenalkylthio mit vorzugsweise 1 bis 4, insbesondere 1 oder 2 Kohlenstoffatomen und 1 bis 5, insbesondere 1 bis 3 Halogenatomen, wie Trifluormethyl, Trifluormethoxy und Trifluormethylthio.

Bevorzugte erfindungsgemäße mikrobizide Mittel enthalten Cyclopropylmethyl(en)ether der Formel

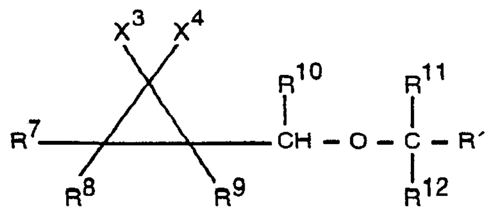

(II),

in der

$X^3$ und $X^4$ gleich oder verschieden sind und für Fluor, Chlor und/oder Brom stehen,

$R^7$ für Wasserstoff oder $C_1$-$C_6$-Alkyl steht,

$R^8$ und $R^9$ gleich oder verschieden sind und für Wasserstoff, $C_1$-$C_6$-Alkyl, $C_1$-$C_4$-Halogenalkyl, 1-Pyrazolylmethyl, 1-Imadazolylmethyl, 1-(1,2,4-Triazolyl)-methyl, $C_1$-$C_4$-Alkoxy-$C_1$-$C_4$-Alkyl, $C_1$-$C_4$-Alkylthio-$C_1$-$C_4$-Alkyl, (Di)$C_1$-$C_4$-Alkylamino-$C_1$-$C_4$-Alkyl oder für gegebenenfalls durch Halogen, wie insbesondere Chlor, Brom oder Jod, substituierte Reste der Reihe Phenyl, $C_2$-$C_4$-Alkenoxy-$C_1$-$C_2$-Alkyl und $C_2$-$C_4$-Alkinoxy-$C_1$-$C_2$-Alkyl stehen,

$R^{10}$ für Wasserstoff oder $C_1$-$C_4$-Alkyl steht,

$R^{11}$ und $R^{12}$ gleich oder verschieden sind und für Wasserstoff, $C_1$-$C_4$-Alkyl oder für gegebenenfalls durch Halogen, wie insbesondere Chlor, Brom oder Jod substituierte Reste der Reihe Phenyl, Benzyl und Phenylethyl stehen, und

R′ für Trijod-$C_2$-$C_4$-alkenyl, Dibrom-jod-$C_2$-$C_4$-alkenyl, Dichlor-jod-$C_2$-$C_4$-alkenyl, $C_2$-$C_4$-Alkinyl oder Jod-$C_2$-$C_4$-Alkinyl steht.

Besonders bevorzugte erfindungsgemäße mikrobizide Mittel enthalten Cyclopropylmethyl(en)ether der Formel

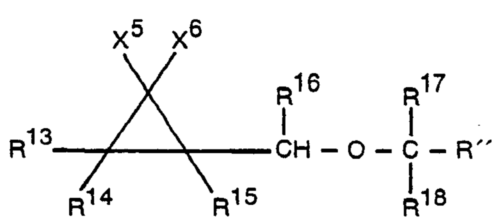

(III),

in der

$X^5$ und $X^6$ gleich oder verschieden sind und für Chlor und/oder Brom stehen,

$R^{13}$ für Wasserstoff, Methyl, Ethyl, n-Propyl oder n-Butyl steht,

$R^{14}$ und $R^{15}$ gleich oder verschieden sind und für Wasserstoff, Methyl, Ethyl, n-Propyl, N-Butyl, 1-Pyrazolylmethyl, 1-Imidazolylmethyl, 1-(1,2,4-Triazolyl)methyl, Methoxymethyl, Ethoxymethyl, Ethoxyethyl, Methylthiomethyl, Ethylthiomethyl, Ethylthioethyl, (Di)Methylaminomethyl, (Di)Methylaminoethyl, (Di)Ethylaminomethyl, (Di)Ethylaminoethyl, Prpenyloxymethyl, Propinyloxymethyl,

Jodpropinyloxymethyl, Trijodpropenyloxymethyl, Dichlorjodpropenyloxymethyl, Dibromjodpropenyloxymethyl, Phenyl, 2-, 3-, 4-Chlorphenyl, 2,4-Dichlorphenyl, 2-, 3-, 4-Bromphenyl, 2,4-Dibromphenyl, Chlormethyl und Brommethyl stehen,

$R^{16}$ für Wasserstoff, Methyl, Ethyl, n- oder i-Propyl steht,

$R^{17}$ und $R^{18}$ gleich oder verscheiden sind und für Wasserstoff, Methyl, Ethyl, n-Propyl, i-Propyl, 2-, 3-, 4-Chlorphenyl, 2,4-Dichlorphenyl oder 4-Bromphenyl stehen und

R'' für Trijodethenyl, Dichlorjodethenyl, Dibromjodethenyl, Jodethinyl oder Jodpropinyl steht.

Insbesonders bevorzugte erfindungsgemäße mikrobizide Mittel enthalten Cyclopropylmethyl(en)ether der Formel

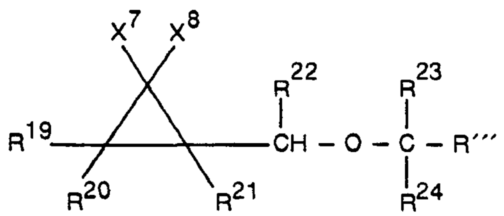

(IV),

in der

$X^7$ und $X^8$ gleich oder verschieden sind und für Chlor und/oder Brom stehen,

$R^{19}$ für Wasserstoff, Methyl oder Ethyl steht,

$R^{20}$ und $R^{21}$ gleich oder verschieden sind und für Wasserstoff, Methyl, Ethyl, Chlormethyl, Brommethyl, 1-Propenyloxymethyl, 1-Propinyloxymethyl oder 1-Jodpropinyloxymethyl stehen,

$R^{22}$ für Wasserstoff oder Methyl steht,

$R^{23}$ und $R^{24}$ gleich oder verschieden sind und für Wasserstoff oder Methyl stehen und

R''' für Trijodethenyl, Dichloriodethenyl, Dibromiodethenyl oder Jodethinyl steht.

Im einzelnen seien die in der folgenden Tabelle angeführten Cyclopropylmethyl(en)ether genannt:

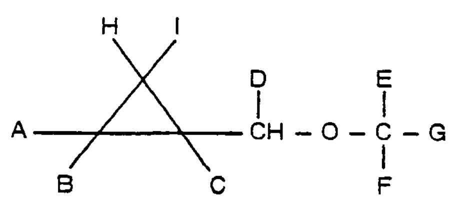

TABELLE

| H | I | A | B | C | D | E | F | G |
|---|---|---|---|---|---|---|---|---|
| Cl | Cl | H | H | H | H | H | H | —C≡CH |
| Cl | Cl | H | H | H | H | H | H | —C≡CI |
| Cl | Cl | H | H | H | H | H | H | —CBr=CBrI |
| Cl | Cl | H | H | H | H | H | H | —CI=$CI_2$ |
| Br | Br | H | $CH_3$ | H | H | H | H | —C≡CH |
| Cl | Cl | H | $CH_3$ | H | H | H | H | —C≡CH |
| Br | Br | H | H | H | H | H | H | —C≡CH |
| Br | Br | H | H | H | H | H | H | —C≡CI |
| Cl | Cl | H | $CH_3$ | H | H | H | H | —C≡CI |
| Cl | Cl | H | H | $CH_3$ | H | H | H | —C≡CI |
| Br | Br | H | $CH_3$ | H | H | H | H | —C≡CI |
| Cl | Cl | H | H | $CH_3$ | H | H | H | —C≡CH |
| Cl | Cl | H | $CH_3$ | H | H | H | H | —C = CI<br>CI CI |
| Br | Br | H | $CH_3$ | H | H | H | H | —C = CI<br>CI CI |
| Br | Br | H | $CH_3$ | H | H | H | H | —CI=$CI_2$ |
| Cl | Cl | H | $CH_3$ | H | H | H | H | —CBr=CBrI |
| Cl | Cl | H | $CH_3$ | H | H | H | H | —CI=$CI_2$ |
| Cl | Cl | H | —$CH_2$Br | H | H | H | H | —C≡CH |
| Cl | Cl | H | —$CH_2OCH_2$C=CH | H | H | H | H | —C≡CH |
| Cl | Cl | H | —$CH_2$Br | H | H | H | H | —C≡CI |
| Cl | Cl | H | —$CH_2OCH_2$C=CI | H | H | H | H | —C≡CI |
| Cl | Cl | H | H | H | $CH_3$ | H | H | —CBr=CBrI |
| Cl | Cl | H | H | $CH_3$ | H | H | H | —CI=$CI_2$ |
| Cl | Cl | H | H | —$CH_2$Cl | H | H | H | —C≡CH |
| Cl | Cl | H | H | $CH_3$ | H | H | H | —C = CI<br>CI CI |

Die erfindungsgemäßen Cyclopropylmethyl(en)ether können hergestellt werden, indem man

a) Cyclopropylmethyl(en)halogenide mit entsprechenden Alkoholen in Gegenwart von Basen wie beispielsweise Natriumhydrid und gegebenenfalls in Gegenwart eines Verdünnungsmittels umsetzt und die auf diese Weise hergestellten Verbindungen mit äquimolarem oder überschüssigem Jod in Gegenwart von z.B. Natronlauge und gegebenenfalls in Gegenwart eines Verdünnungsmittels umsetzt oder

b) die nach Absatz (a) hergestellten Verbindungen mit Halogenen, wie Chlor, Brom oder Jod, gegebenenfalls in Gegenwart eines Verdünnungsmittels umsetzt.

Technische Materialien im Rahmen der vorliegenden Erfindung sind Produkte, die selbst nicht in der Natur vorkommen, sondern aus natürlichen oder synthetischen Ausgangsmaterialien gefertigt werden. Die zu schützenden Produkte im Rahmen der vorliegenden Erfindung sind technische Materialien, die durch Mikroorganismen zersetzt werden können.

Technische Materialien, die durch die erfindungsgemäßen Substanzen vor einer mikrobiellen Veränderung und Zerstörung geschützt werden sollen, sind beispielsweise Klebstoffe, Leime, Papiere und Kartone, Textilien, Leder, Holz, Anstrichmittel, Putze, Kühlschmiermittel und Kunststoffartikel, die von Mikroorganismen befallen und zersetzt werden können. Im Rahmen der zu schützenden Materialien seien auch Teile von Produktionsanlagen, wie beispielsweise Kühlwasser- und Kühlschmiermittelkreisläufe genannt, deren Funktionstüchtigkeit durch Mikroorganismen beeinträchtigt werden kann. Vorzugsweise können die erfindungsgemäßen Wirkstoffe zum Schutz von Klebstoffen, Papier, Karton, Anstrichfilmen, Holz u.ä. verwendet werden.

Mikroorganismen, die einen Abbau oder eine Veränderung der technischen Materialien bewirken können, sind beispielsweise Bakterien, Pilze, Hefen, Algen und Schleimeorganismen.

Vorzugsweise wirken die erfindungsgemäßen Substanzen gegen Pilze und Schleimorganismen; von der fungiziden Wirkung werden Schimmelpilze ebenso erfaßt wie Holzzerstörende und Holz-verfärbende Pilze.

Es seien beispielsweise Mikroorganismen der folgenden Gattungen gennant:

Escherichia, wie Escherichia coli,
Pseudomonas, wie Pseudomonas aeruginosa,
Staphylococcus, wie Staphylococcus aureus,
Alternaria, wie Alternaria tenuis,
Aspergillus, wie Aspergillus niger,
Aureobasidium, wie Aureobasidium pullulans,
Chaetomium, wie Chaetomium globosum,
Coniophora, wie Coniophora cerebella,
Lentinus, wie Lentinus tigrinus,
Penicillium, wie Penicillium glaucum,
Polyporus, wie Polyporus versicolor,
Sclerophoma, wie Sclerophoma pityophila,
Trichoderma, wie Trichoderma viride.

Je nach ihrem Anwendungsgebiet können die erfindungsgemäßen Substanzen in die üblichen Formulierungen überführt werden, wie Lösungen, Emulsionen, Suspensionen, Pulver, Pasten und Granulate. Diese können in an sich bekannter Weise hergestellt werden, z.B. durch Vermischen der Wirkstoffe mit einem Streckmittel, also flüssigem Lösungsmittel und/oder festen Trägerstoffen, gegebenenfalls unter Verwendung von oberflächenaktiven Mitteln, wie Emulgatoren und/oder Dispergiermitteln, wobei beispielsweise im Falle der Benutzung von Verstreckmitteln, gegebenenfalls organische Solventien, als Hilfslösungsmittel verwendet werden können.

Organische Solventien für die Wirkstoffe können beispielsweise Alkohole, wie niedere Alkohole, vorzugsweise Ethanol oder Isopropanol, oder Benzylalkohol, Ketone wie Aceton oder Methylethylketon, flüssige Kohlenwasserstoffe, wie Benzinfraktionen, chlorierte Kohlenwasserstoffe, wie 1,2-Dichloroethan sein.

Die erfindungsgemäßen mikrobiziden Mittel enthalten im allgemeinen 10 bis 100 Gew.-%, bevorzugt 50 bis 80 Gew.-% der substituierten Cyclopropylmethyl(en)ether als Wirkstoff.

Die Anwendungskonzentration der erfindungsgemäßen Substanzen richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 0,5 Gew.-%, bezogen auf das zu schützende Material.

Die erfindungsgemäßen neuen Wirkstoffe können auch in Mischung mit anderen bekannten Wirkstoffen vorliegen. Beispielsweise seien die folgenden Wirkstoffe genannt:

Benzimadazolyl-methylcarbamate, Tetramethylthiuramdisulfid, N-Fluordichlormethylthio-phthalimid und N,N - Dimethyl - N' - phenyl - (N' - fluordichlormethylthio) - sulfamid, Formaldehyd - abspaltende

Verbindungen, wie Hemiformale, Oxazolidine, Hexahydro-s-triazine, N-Methylolamide und Phenolderivate, wie p-Chlor-m-kresol, 2-Phenyl-phenol, (2,2'-Dihydroxy-5,5'-dichlor)-diphenylmethan.

Die erfindungsgemäßen mikrobiziden Mittel, die als Wirkstoff Cyclopropylmethyl(en)ether enthalten, haben überraschenderweise eine höhere spezifische Wirkung als vergleichbare Mittel. Sie zeichnen sich besonders durch niedrige Anwendungskonzentration aus.

Herstellungsbeispiele:

Beispiel 1:

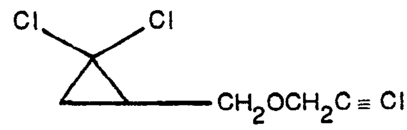

(Verfahren a)

4,5 g (0,025 Mol) 3-(2,2-Dichlorcyclopropylmethoxy)-1-propin werden in 100 ml Methanol gelöst und bei 20°C portionsweise und im stetigen Wechsel mit 6,3 g (0,025 Mol) Jod und 2,5 g 45 %-iger Natronlauge versetzt. Nach der Zugabe wird noch 1 Stunde bei 20°C gerührt. Nach Abdestillieren des Methanols wird der Rückstand in 100 ml Dichlormethan aufgenommen, mit 50 ml verdünnter Natriumthiosulfatlösung und anschließend mit 50 ml Wasser gewaschen. Die organische Phase wird über Magnesiumsulfat getrocknet.

Man erhält nach Abdestillieren flüchtiger Bestandteile bei 1 mbar und 95°C als Rückstand 3,8 g (50 % der Theorie) 3-(2,2-Dichlorcyclopropylmethoxy)-1jod-1-propin vom Brechungsindex $n_D^{20}$ : 1,5818.

Beispiel 2:

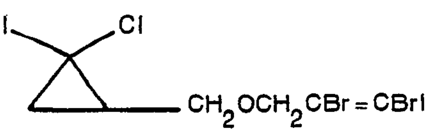

(Verfahren b)

3 g (0,01 Mol); 3-(2,2-Dichlorcyclopropylmethoxy)-1-jod-1-propin werden in 50 ml Methylenchlorid gelöst und bei 20°C mit 2 g Brom (0,0125 Mol) in 20 ml Methylenchlorid versetzt. Anschließend wird 14 Stunden bei 60°C gerührt. Nach Abkühlung auf 20°C wird das Gemisch mit 20 ml wäßriger Natriumthiosulfatlösung und dann mit Wasser gewaschen. Die organische Phase wird über Magnesiumsulfat getrocknet und das Lösungsmittel im Wasserstrahlvakuum entfernt.

Man erhält 4,2 g (90 % der Theorie) 1,2-Dibrom-3-(2,2-dichlorcyclopropylmethoxy)-1-jod-1-propen vom Brechungsindex $n_D^{20}$ : 1,6014.

Beispiel 3

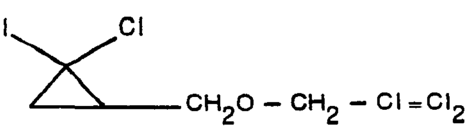

(Verfahren a)

23 g (0,128 Mol) 3-(2,2-Dichlorcyclopropylmethoxy)-1-propin in 100 ml Methanol werden bei 20°C unter Rühren portionsweise und im stetigen Wechsel mit 96 g (0,378 Mol) Jod und 13 g 45 %-iger Natronlauge versetzt und 14 Stunden gerührt. Das Lösungsmittel wird anschleißend im Wasserstrahlvakuum abdestilliert und der Rückstand mit 500 ml Methylenchlorid versetzt. Bis zur Entfärbung wird zunächst mit 200 ml verdünnter Natriumthiosulfatlösung und dann mit 200 ml Wasser gewaschen. Die organische Phase wird über Magnesiumsulfat getrocknet und im Wasserstrahlvakuum eingeengt.

Man erhält 46 g (64 % der Theorie) 3-(2,2-Dichlorcyclopropylmethoxy)-1,1,2-trijod-1-propen vom Brechungsindex: $n_D^{20}$ : 1,6623.

Analog Verfahrensvariante (a) bzw. (b) können die in der nachstehenden Tabelle 1 aufgeführten Verbindungen der Formel (I) hergestellt werden:

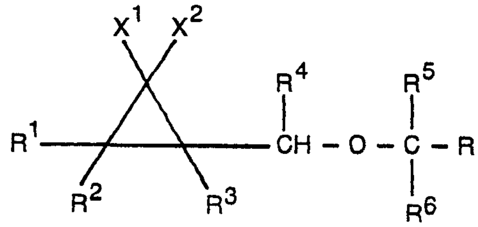

(I)

TABELLE 1

| Beisp.-Nr. | $X^1$ | $X^2$ | $R^1$ | $R^2$ | $R^3$ | $R^4$ | $R^5$ | $R^6$ | R | $n_D^{20}$ |
|---|---|---|---|---|---|---|---|---|---|---|
| 4 | Br | Br | H | H | H | H | H | H | —C≡CI | 1,5980 |
| 5 | Cl | Cl | H | $CH_3$ | H | H | H | H | —C≡CI | 1,5540 |
| 6 | Cl | Cl | H | H | $CH_3$ | H | H | H | —C≡CI | 1,5530 |
| 7 | Br | Br | H | $CH_3$ | H | H | H | H | —C≡CI | 1,5620 |
| 8 | Cl | Cl | H | $CH_3$ | H | H | H | H | —C=CI<br>Cl Cl | 1,5484 |
| 9 | Br | Br | H | $CH_3$ | H | H | H | H | —C=CI<br>Cl Cl | 1,5780 |
| 10 | Br | Br | H | $CH_3$ | H | H | H | H | $—CI=CI_2$ | 1,6496 |
| 11 | Cl | Cl | H | $CH_3$ | H | H | H | H | —CBr=CBrI | 1,5920 |
| 12 | Cl | Cl | H | CH3 | H | H | H | H | $—CI=CI_2$ | 1,6396 |
| 13 | Cl | Cl | H | $—CH_2Br$ | H | H | H | H | —C≡CI | 1,5565 |
| 14 | Cl | Cl | H | $—CH_2OCH_2C≡CI$ | H | H | H | H | —C≡CI | 1,5920 |
| 15 | Cl | Cl | H | H | $CH_3$ | H | H | H | —CBr=CBrI | 1,5391 |
| 16 | Cl | Cl | H | H | $CH_3$ | H | H | H | $—CI=CI_2$ | 1,6464 |
| 17 | Cl | Cl | H | H | $CH_3$ | H | H | H | —C=CI<br>Cl Cl | 1,5547 |

Anwendungsbeispiele:

Beispiel 19: Wirkung gegen Pilze

In einen Agar, der aus Bierwürze und Pepton hergestellt wurde, wurden die erfindungsgemäßen Verbindungen in abgestuften Konzentrationen zwischen 1 und 5000 mg/l Versuchsprobe eingearbeitet. Nach dem Erstarren des Agars erfolgte die Kontamination der so hergestellten Agarproben mit Reinkulturen verschiedener Testpilze (siehe Tabelle A).

Nach zweiwöchiger Lagerung bei 28°C und 60 bis 70 % relativer Luftfeuchtigkeit wurde ausgewertet. In der Tabelle ist als minimale Hemmkonzentration (MHK) die geringste in einer Agarprobe enthaltene Konzentration der Substanz angegeben, bei der keinerlei Bewuchs durch die verwendete Art erfolgte.

In diesem Test zeigen z.B. folgende Verbindungen eine sehr gute Wirkung:

Verbindungen gemäß Herstellungsbeispielen 1, 5, 6, 7, 11, 12, 3, 15 und 16.

TABELLE A: Wirkung gegen Pilze (MHK-Werte in mg/l für folgende Spezies)

| | 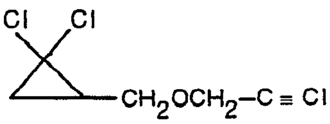 (1) | 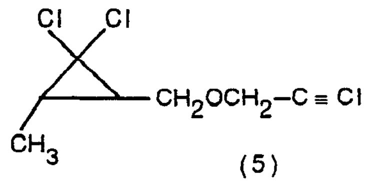 (5) | 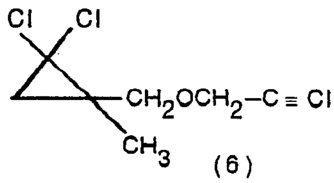 (6) |
|---|---|---|---|
| Alternaria tenuis | 2 | 5 | 35 |
| Aspergillus niger | 1 | 2 | 5 |
| Aureobasidium pullulans | 1 | 3 | 5 |
| Chaetomium globosum | 75 | 75 | 100 |
| Coniphora cerebella | 0,1 | 0,5 | 0,75 |
| Lentinus tigrinus | 2 | 5 | 7,5 |
| Penicillium glaucum | 15 | 2 | 5 |
| Polyporus versicolor | 1 | 2 | 2 |
| Sclerophoma pityophila | 2 | 5 | 5 |
| Trichoderma viride | 10 | 20 | 20 |

TABELLE A — Fortsetzung:

| | 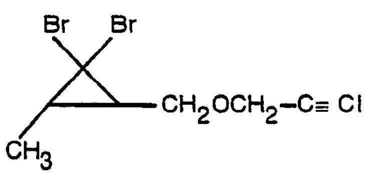 (7) | 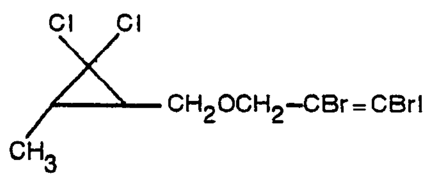 (11) | 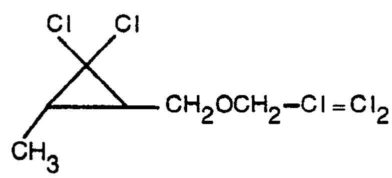 (12) |
|---|---|---|---|
| Alternaria tenuis | 5 | 200 | — |
| Aspergillus niger | 5 | 500 | 50 |
| Aureobasidium pullulans | 5 | 100 | — |
| Chaetomium globosum | 50 | 350 | 50 |
| Coniphora cerebella | 0,75 | 20 | — |
| Lentinus tigrinus | 10 | 75 | — |
| Penicillium glaucum | 5 | 200 | 50 |
| Polyporus versicolor | 5 | 100 | — |
| Sclerophoma pityophila | 10 | 100 | — |
| Trichoderma viride | 50 | 500 | — |

TABELLE A — Fortsetzung

| | 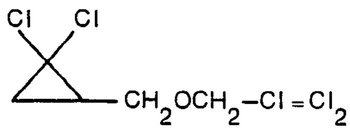 (3) | 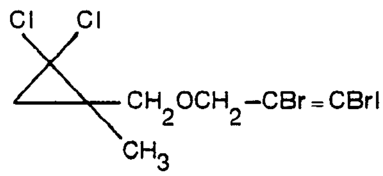 (15) |
|---|---|---|
| Alternaria tenuis | — | — |
| Aspergillus niger | <20 | 100 |
| Aureobasidium pullulans | — | — |
| Chaetomium globosum | <20 | 100 |
| Coniphora cerebella | — | — |
| Lentinus tigrinus | — | — |
| Penicillium glaucum | <20 | 100 |
| Polyporus versicolor | — | — |
| Sclerophoma pityophila | — | — |
| Trichoderma viride | — | — |

TABELLE A — Fortsetzung

| | 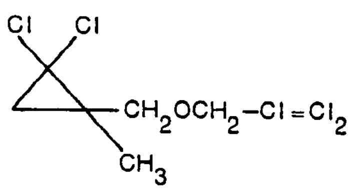 (16) | 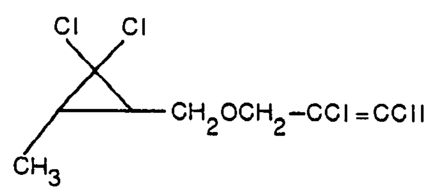 (8) |
|---|---|---|
| Alternaria tenuis | — | — |
| Aspergillus niger | <20 | <20 |
| Aureobasidium pullulans | — | — |
| Chaetomium globosum | 35 | <20 |
| Coniphora cerebella | — | — |
| Lentinus tigrinus | — | — |
| Penicillium glaucum | <20 | <20 |
| Polyporus versicolor | — | — |
| Sclerophoma pityophila | — | — |
| Trichoderma viride | — | — |

Beispiel 20: Wirkung gegen Schleimorganismen

Erfindungsgemäß Verbindungen werden in Konzentrationen von jeweils 0,1 bis 100 mg/l in Allens Nährlösung (Arch. Mikrobiol. *17*, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus der bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbeleibt.

In diesem Test zeigen z.B. folgende Verbindungen eine sehr gute Wirkung:

Verbindungen gemäß Herstellungsbeispielen 1, 5, 6 und 7.

TABELLE B

Wirkung gegen Schleimorganismen

| Wirkstoff | MHK-Werte in mg / l bei der Einwirkung auf Schleimorganismen |
|---|---|
| 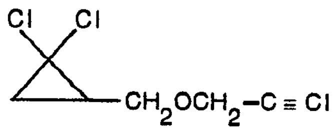 (1) | 5 – 10 |
| 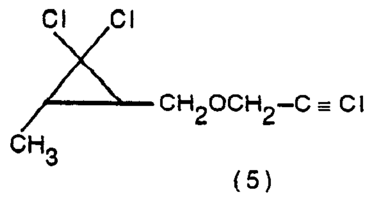 (5) | 5 – 10 |
| 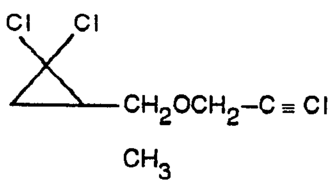 (6) | 5 – 10 |
| 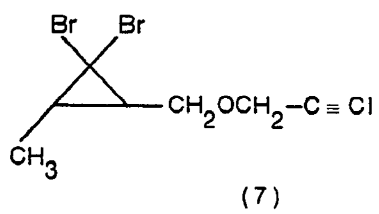 (7) | 10 |

**Patentansprüche**

1. Mikrobizides Mittel zum Schutz technischer Materialien, enthaltend Cyclopropylmethyl(en)ether der Formel

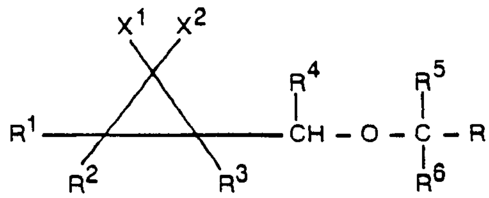

(I),

in der

$X^1$ und $X^2$ gleich oder verschieden sind und für Halogen stehen,

$R^1$ für Wasserstoff oder Alkyl steht,

$R^2$ und $R^3$ gleich oder verschieden sind und für Wasserstoff, Alkyl, gegebenenfalls substituiertes Aryl, Halogenalkyl, Hetaryl-alkyl, Alkoxyalkyl, Alkylthioalkyl, gegebenenfalls substituiertes Alkenoxyalkyl, gegebenenfalls substituiertes Alkinoxyalkyl oder für (Di)Alkylaminoalkyl stehen,

$R^4$ für Wasserstoff oder Alkyl steht,

$R^5$ und $R^6$ gleich oder verschieden sind und für Wasserstoff oder gegebenenfalls substituierte Reste aus der Reihe Alkyl, Aryl und Aralkyl stehen und

R für einen Trihalogenalkenyl-, Alkinyl-, oder Jodalkinyl-Rest steht.

2. Mikrobizide Mittel nach Anspruch 1, enthaltend 10 bis 100 Gew.-% der Cyclopropylmethyl(en)ether.

3. Verwendung von Cyclopropylmethyl(en)ether der Formel

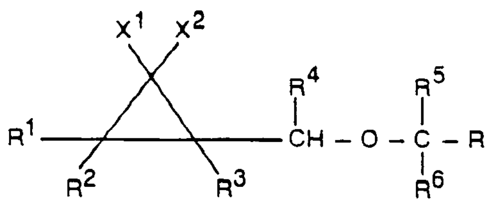

(I),

zum Schutz technischer Materialien vor Schädigung oder Zerstörung durch Mikroorganismer.

4. Verwendung nach Anspruch 3 zur Bekämpfung von Pilzen an technischen Materialien.

**Revendications**

1. Agent microbicide pour la protection de matières techniques, cet agent contenant des éthers cyclopropylméthyliques(méthyléniques) de formule:

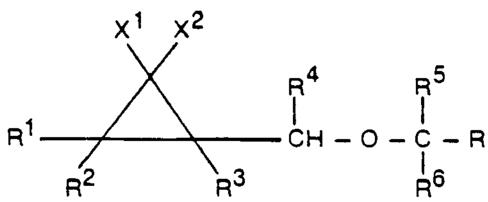

(I)

dans laquelle

$X^1$ et $X^2$ sont identiques ou différents et représentent chacun un atome d'halogène,

$R^1$ représente un atome d'hydrogène ou un groupe alkyle,

$R^2$ et $R^3$ sont identiques ou différrents et représentent chacun un atome d'hydrogène, un groupe alkyle, un groupe aryle éventuellement substitué, un groupe halogénalkyle, un groupe hétarylalkyle, un groupe alcoxyalkyle, un groupe alkylthioalkyle, un groupe alcénoxyalkyle éventuellement substitué, un groupe alcynoxyalkyle éventuellement substitué ou un groupe (di)alkylaminoalkyle,

$R^4$ représente un atome d'hydrogène ou un groupe alkyle,

$R^5$ et $R^6$ sont identiques ou différents et représentent chacun un atome d'hydrogène ou des radicaux éventuellement substitués choisis parmi la série comprenant les groupes alkyle, aryle et aralkyle, et

R représente un groupe trihalogénalcényle, un groupe alcynyle ou un groupe iodalcynyle.

2. Agents microbicides selon la revendication 1 contenant 10 à 100% en poids des éthers cyclopropylméthyliques(méthyléniques).

3. Utilisation d' éthers cyclopropylméthyliques(méthyléniques) de formule:

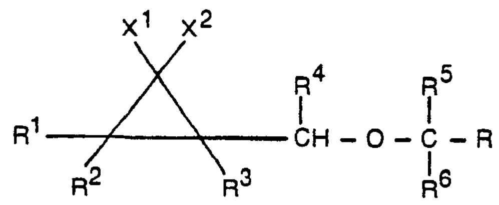

(I)

pour la protection de matières techniques contre la détérioration ou la destruction par les micro-organismes.

4. Utilisation selon la revendication 3 pour combattre les champignons sur les matières techniques.

**Claims**

1. Micobicidal agent for protecting industrial materials, containing cyclopropylmethyl(ene) ethers of the formula

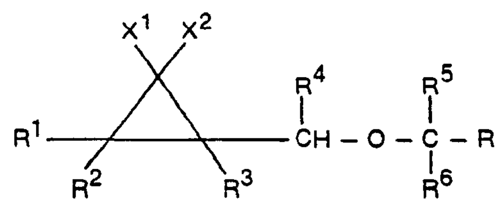

(I)

in which

$X^1$ and $X^2$ are identical or different and represent halogen,

$R^1$ represents hydrogen or alkyl,

$R^2$ and $R^3$ are identical or different and represent hydrogen, alkyl, optionally substituted aryl, halogenoalkyl, hetaryl-alkyl, alkoxyalkyl, alkylthioalkyl, optionally substituted alkenoxyalkyl, optionally substituted alkinoxyalkyl or (di)alkylaminoalkyl,

$R^4$ represents hydrogen or alkyl,

$R^5$ and $R^6$ are identical or different and represent hydrogen or optionally substituted radicals from the series comprising alkyl, aryl and aralkyl and

R represents a trihalogenoalkenyl, alkinyl or iodoalkinyl radical.

2. Microbicidal agents according to Claim 1, containing 10 to 100% by weight of the cyclopropylmethyl(ene) ethers.

3. Use of cyclopropylmethyl(ene) ethers of the formula

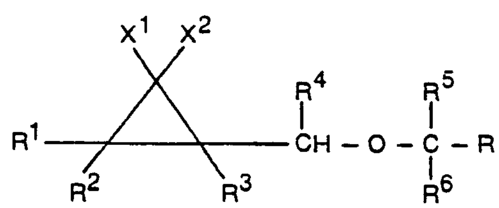

(I)

for protecting industrial materials against damage or destruction by microorganisms.

4. Use according to Claim 3 for combating fungi on industrial materials.